# EUROPEAN PATENT SPECIFICATION

(11) **EP 3 383 269 B1**
(45) Date of publication and mention of the grant of the patent: **19.02.2020**
(21) Application number: 16802145.9
(22) Date of filing: 17.11.2016
(51) Int. Cl.: A61M 5/19, A61B 5/15, A61B 5/153, B04B 5/04

(54) **APPARATUS FOR PREPARING BLOOD FRACTION CONCENTRATE**
VORRICHTUNG ZUR HERSTELLUNG EINES BLUTFRAKTIONSKONZENTRATS
APPAREIL DE PRÉPARATION DE CONCENTRÉ DE FRACTION DE SANG

(30) Priority: 03.12.2015 EP 15197876
(43) Date of publication of application: 10.10.2018
(73) Proprietor: Lacerta Technologies Inc., Raleigh NC 27616-6417 (US)
(72) Inventor: LACZA, Zsombor, 8229 Csopak (HU); HORNYÁK, István, 3530 Miskolc (HU)
(74) Representative: Danubia Patent & Law Office LLC
(86) International application number: PCT/IB2016/056933
(87) International publication number: WO 2017/093838

(56) References cited:
- WO-A1-2006/122400
- WO-A1-2014/160781
- US-A- 5 139 031
- US-A- 5 738 784
- US-A1- 2013 011 311

## Description

### Field of the Invention

The invention relates to preparation of blood fractions.

Blood can be fractionated, and the different fractions of the blood are useful for different medical needs. Under the influence of gravity or centrifugal force, blood can separate into two or more layers. Also, some blood constituents may also be enriched selectively, induced by activating selective biologically active reservoirs or allowing blood constituents to react and convert into altered molecules.

### Prior Art Discussion

WO2014/126970 (Lacerta Technologies Inc.) describes methods of preparing isolated serum fractions. A platelet rich plasma ("PRP") is provided without addition of an anticoagulant. This is clotted to provide a coagel (clot) of a platelet rich fibrin ("PRF"). The coagel is a jelly-like mass resulting from conversion of fibrinogen to fibrin, mainly fibrin fibres associated to form a fibrin gel or clot. These two steps can be done simultaneously by, for example, centrifuging.

US2013/0343967 (Lacerta Technologies Inc) describes a device providing a closed system for centrifuging tissue aspirates. It has a syringe with a cannula which is inserted into a container. US4957637 (Sherwood Medical Company) describes a serum separator system for centrifuge, having a pierceable membrane.

EP2898909 (Arthrex Inc) describes a triple syringe.

WO2014/160781 (Alliance Partners) describes a biological fluids concentration device with a tube-in-tube assembly, for centrifuging.

The invention is directed towards providing an improved apparatus for preparation of blood fractions, for applications including those of WO2014/126970.

### SUMMARY OF THE INVENTION

According to the invention, there is provided a blood handling device comprising: a syringe with a syringe tube for taking a blood sample, and a container releasably connected to the syringe, with a chamber and a fluid passageway between the syringe and the chamber, a connector interfacing between the syringe and the container, said connector having said fluid passageway, and said connector comprises a first part forming a base for the syringe, and a second part arranged to connect to the container or to a cannula, wherein the syringe tube and the container have an inner wall configured to activate fibrinogen to fibrin conversion, and thus to separate blood fractions and to form a fibrin clot upon contacting said blood sample with said tube and said container during centrifugation.

In one embodiment, the syringe has a plunger releasably connected to a piston within a tube. In one embodiment, the plunger and the piston have inter-engaging screw threads.

In one embodiment, there is a gap between said connector parts, and optionally said gap is in the range of 0.1 mm and 7 mm. preferably, the connector first part comprises a plate with a surface facing into the syringe volume. In one embodiment, the plate is dish-shaped with a generally concave surface facing into the syringe volume.

In one embodiment, said plate has an aperture aligned with a passageway in the container second part. Preferably, said passageway is elongate. In one embodiment, a gap is maintained by spacers of one part engaging in apertures of the other part.

In one embodiment, the connector first and second parts are releasably engaged.

Preferably, the connector second part comprises a luer fastener suitable for engaging a cannula or the container.

In one embodiment, the container has a piston fitted to move in the chamber in a direction away from the syringe under applied forces during centrifuging of the device. Preferably, the chamber piston has a lower stem arranged to move axially within a guide passageway in the container. In one embodiment, the container has a resilient seal around said guide passageway at a bottom wall of the chamber.

In one embodiment, the width of the passageway is between 0.5 mm and 6 mm and the length of the passageway is between 8 mm and 30 mm.

In one embodiment, the device includes a hydrophilic blood-contacting material such as hydroxyapatite or a polymer with an ionic surface modification.

Also described (not claimed) is a method of handing blood using a device comprising a syringe for taking a blood sample, and a container with a chamber releasably connected to the syringe, and a fluid passageway between the syringe and the chamber, a connector interfacing between the syringe and the container, said connector having said fluid passageway and said connector comprises a first part forming a base for the syringe, and a second part arranged to connect to the container or to a cannula, the method comprising the steps of:
taking a blood sample in the syringe when the syringe is disconnected from the container,
fastening the container to the syringe,
centrifuging the assembly of the syringe and the container,
removing the container from the syringe, leaving a first blood fraction within the syringe and a second blood fraction within the container.

In one example, the method comprises the further step of injecting the first blood fraction from the syringe to a patient.

In one example, the centrifuging step results in a clot forming on an inner surface of the device. In one embodiment, the clot is formed on a surface around said fluid passageway.

In one example, there is a space surrounding at least part of the passageway, and an additive is placed in said space before centrifuging. In one embodiment, said additive comprises reactive particles such as titanium beads or glass beads.

In one example, the centrifugal force is between 5000 m/s² and 50000 m/s², preferably between 10000 m/s² and 30000 m/s², and more preferably between 15000 m/s² and 20000 m/s².

In one example, the time of the centrifugation is between 3 and 30 minutes, preferably between 5 and 15 minutes, and more preferably between 5 and 10 minutes.

In one example, the method takes place within 1 to 60 minutes, preferably between 3 and 30 minutes, more preferably between 5 and 10 minutes after drawing the blood from the patient.

### Additional Statements

According to the invention, there is provided a blood handling device as defined in claim 1.

### DETAILED DESCRIPTION OF THE INVENTION

### Brief Description of the Drawings

The invention will be more clearly understood from the following description of some embodiments thereof, given by way of example only with reference to the accompanying drawings in which:-
Figs. 1 and 2 are perspective and elevation views of a device of the invention for preparation and use of blood fractions;
Fig. 3 is a perspective view with an outer casing removed;
Fig. 4 is an exploded perspective view with the casing and syringe tube removed;
Fig. 5 is a top plan, and Fig. 6 is an underneath plan of the device;
Fig. 7 is a cross-sectional view showing internal construction in more detail;
Fig. 8 is a perspective cut-away view of a plunger, and Fig. 9 is a full perspective view of the plunger;
Fig. 10 is cut-away perspective view of a syringe tube piston and base top part, and Fig. 11 are elevation cut-away views;
Figs. 12 and 13 are cross-sectional and cut-away perspective views respectively of the container;
Figs. 14 and 15 are diagrammatic cross-sectional views showing the syringe piston in different positions;
Figs. 16 and 17 are diagrammatic cross-sectional views showing the syringe tube connector connected to the container, and Fig. 18 is a perspective cut-away view;
Figs. 19 to 23 are a series of diagrams showing the device in use; and
Figs. 24 and 25 are cross-sectional views of an alternative container of the device.

### Description of the Embodiments

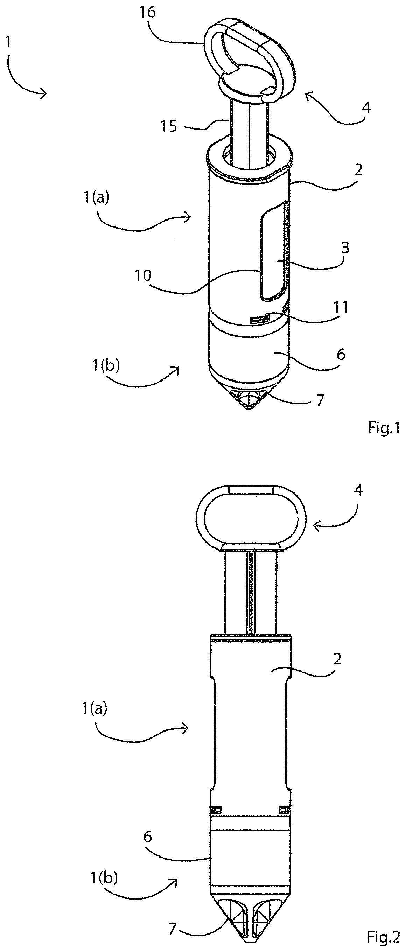

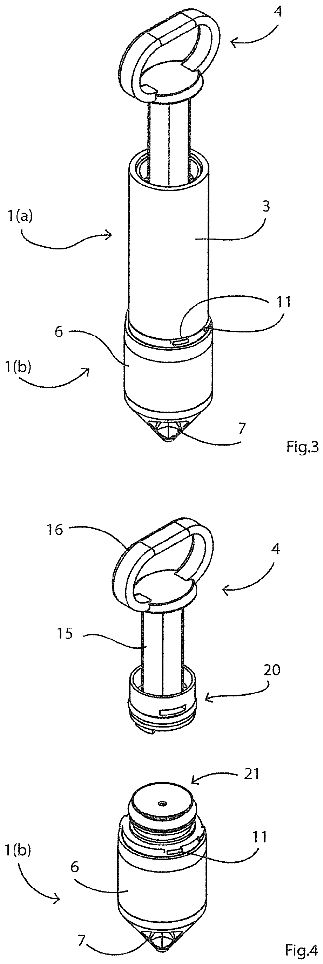

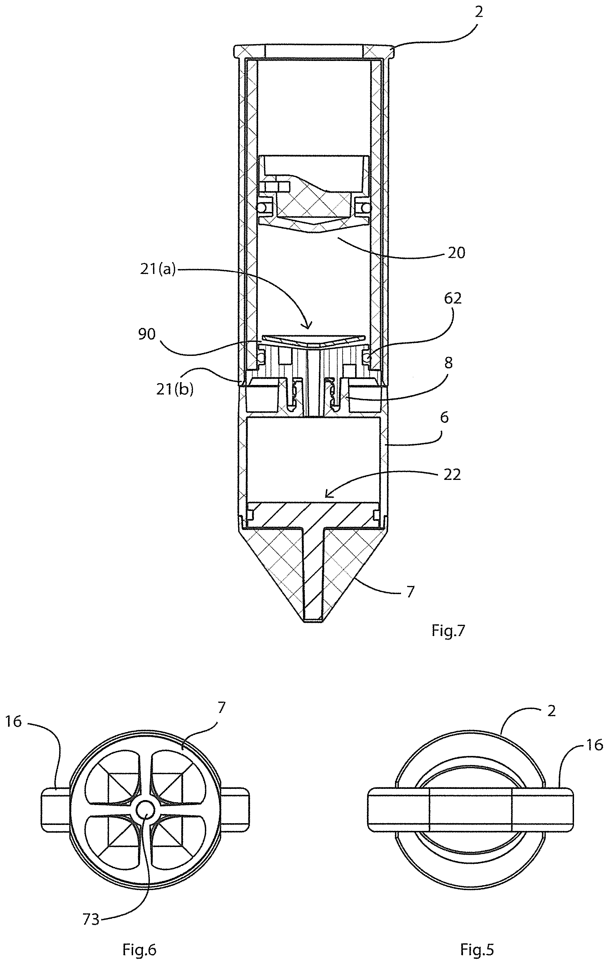

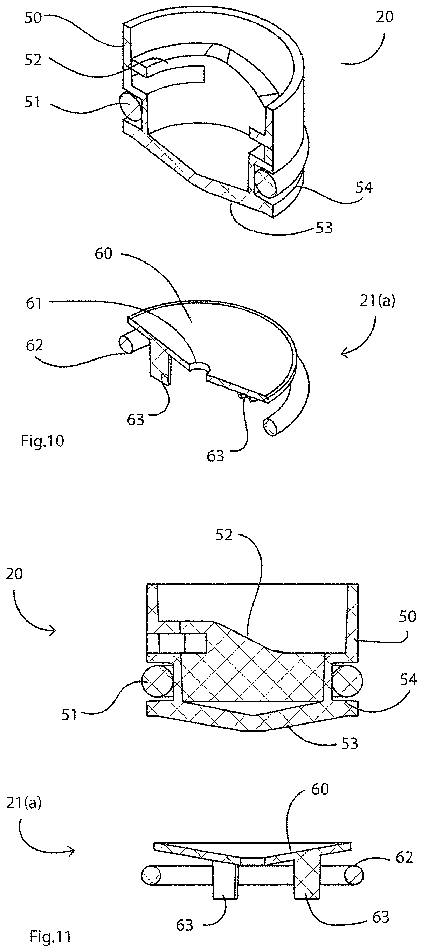

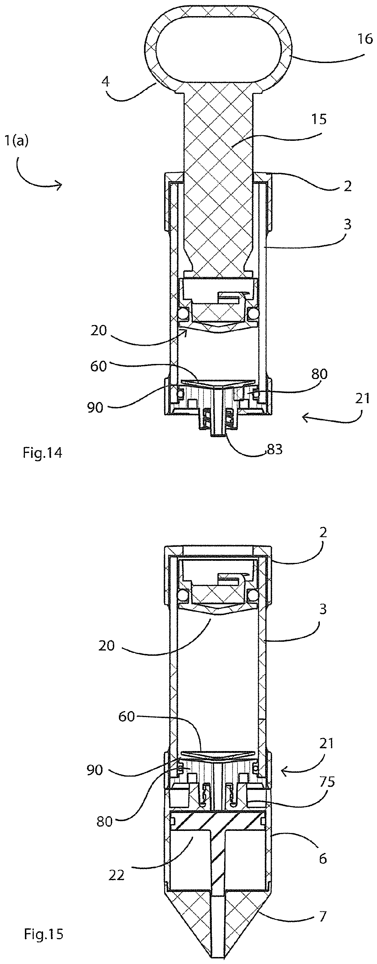

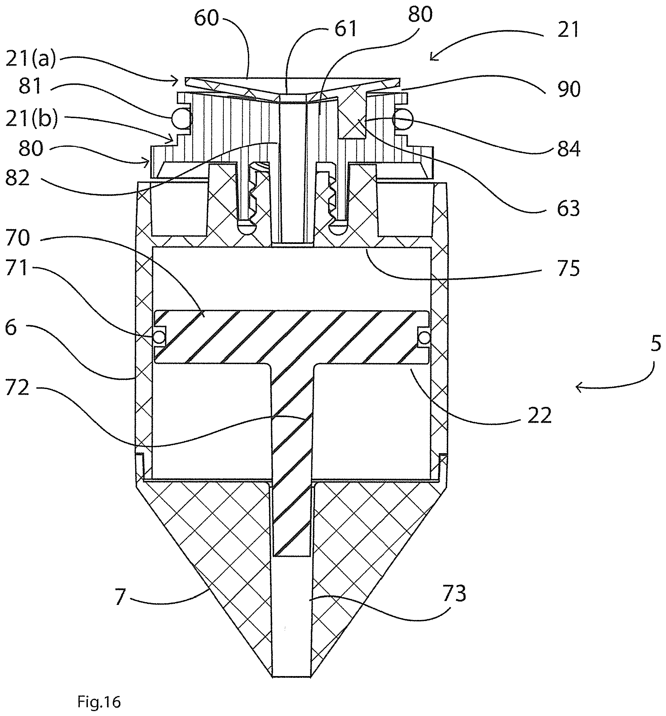

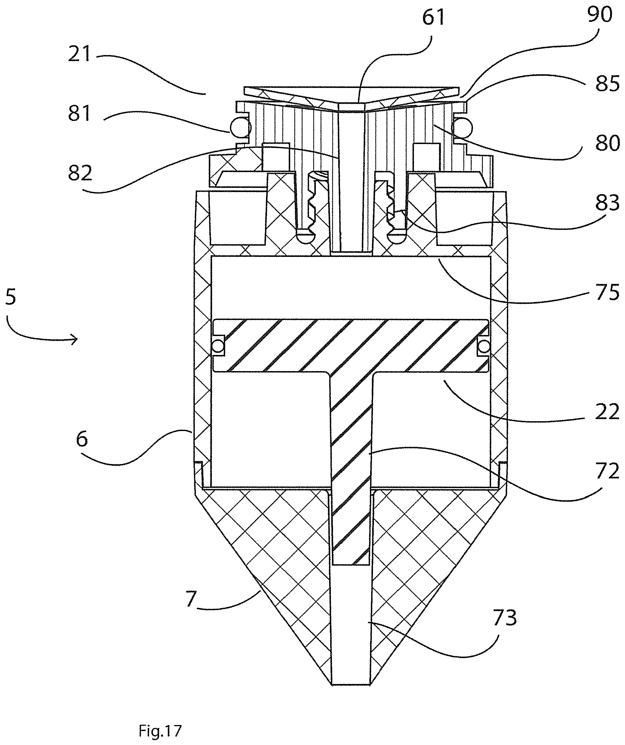

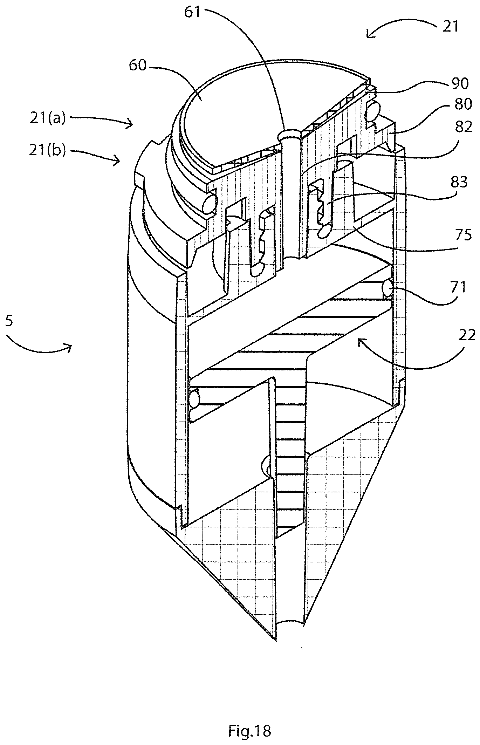

Referring to Figs. 1 to 18, and especially initially to Figs. 1 to 7, a device 1 comprises a syringe 1(a) with an outer casing 2 around a transparent syringe tube 3 (viewable through elongate windows 10 of the casing 2). A plunger 4 comprising a stem 15 and a handle 16 extends into the syringe tube 3. Beneath the syringe 1(a) there is a detachable container 1(b) having a chamber 6 and a cone 7.

The plunger 4 is removably attached to a piston 20 within the syringe tube 3. The lower end of the tube 3 has a removable base formed by a connector 21 with first and second interconnecting parts 21(a) and 21(b) respectively. The latter has radial tabs 11 for snap-fitting into slots in the syringe outer casing 2, to retain the outer casing over the syringe tube 3.

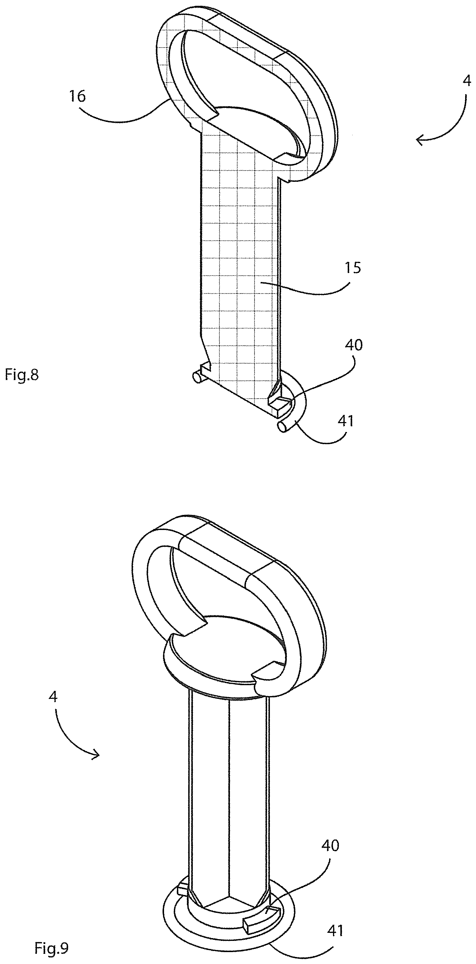

Referring especially to Figs. 8 to 11, the plunger 4 comprises external threads 40 for releasably engaging the piston 20. There is also a sealing O-ring 41 for sealing against the inside surface of the syringe tube 3.

The piston 20 comprises a generally tubular wall 50 forming a circumferential groove 54 for an O-ring 51, and an internal thread 52 to removably engage the plunger stem's external thread 40. The surface of the piston 20 facing into the syringe tube 3 is formed by a large-angle conical wall 53 facing into the syringe tube 3.

The connector first part 21(a) comprises a dish-shaped plate 60 having a central aperture 61. It seals with the syringe tube 3 by an O-ring 62. There are also three equally radially-spaced and axially-extending spacers 63 for engaging the lower part 21(b). These pins or spacers 63 act to maintain a gap 90 between the connector parts 21(a) and 21(b), as described in more detail below.

The syringe 1(a) is linked by a fluidic passageway in the connector 21 to the container 1(b), as described in more detail below.

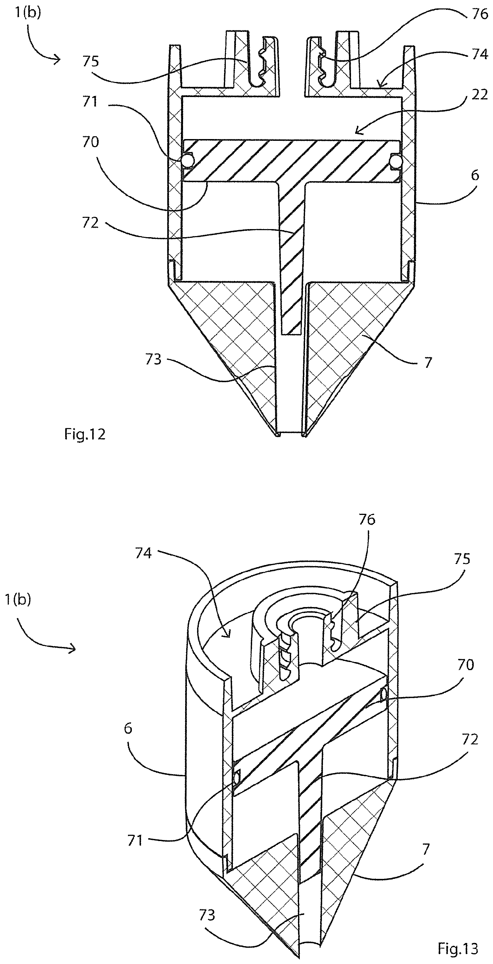

Referring especially to Figs. 12 to 18, the container piston 22 has a disc 70 with an O-ring 71, and a downwardly-depending stem 72 extending through an axial passageway 73 in the cone 7.

There is no actuator for the piston 22, it moves during centrifuging as a result of the externally-applied centrifuging forces.

The container chamber 6 has an integral cover 74 with a socket 75 having external threads 76 to engage the connector 21.

The connector part 21(b) comprises a body 80 with blind apertures 84 to receive the spacers 63, a groove for an O-ring 81 sealing against the syringe tube 3, and a elongate central through passageway 82. The body 80 also has a tubular protrusion 83 with internal threads for engaging the corresponding external threads 76 of a socket 75 of the chamber 6. The passageway 82 extends through the body 80 so that when the protrusion 83 is screwed to the threads 76 of the chamber 6 the passageway 82 terminates in the chamber 6. The protrusion 83 is in the configuration of a luer fastener.

Hence, a conduit linking the volume within the syringe tube 3 and the chamber 6 is formed by the aperture 61, the gap 90, and the passageway 82.

The plunger 4 is detachable, as is the container 1(b). The piston 20 seals and allows application of pressure and vacuum within the tube 3. The other end of the tube 3 is fitted with the connector 21, which seals and connects to the container chamber 6. The piston 22 seals and moves within the chamber 6. Importantly, the spacers 63 and the blind apertures 84 maintain the gap 90 between the connector parts 21(a) and 21(b).

The total length of the device without the plunger 4 is between 80 and 140 mm, preferably between 100 and 120 mm, more preferably between 105 and 120 mm. The device is compatible with general swing-out rotors, which are suitable for 50 ml conical centrifuge vials.

In general terms the length of the conduit (61, 90, 82) from the syringe to the container is preferably in the range of 8 mm to about 30 mm in total. The diameter of the opening 61 in the connector dish 60 is between 2 mm and 3 mm. The length and diameter of the passageway 82 are between 8 mm and 20 mm and between 0.5 mm and 6 mm. The separation 90 between the dish 61 and the top surface 85 of the connector second part 21(b) is between 0.1 mm to 7 mm. In summary, the dimensions of the conduits and openings linking the syringe 1(a) to the container 1(b) may be in the ranges of:

| | |
|---|---|
| dish 61 diameter: | 6 mm to 22 mm, |
| separation 90: | 0.1 mm to 7 mm, |
| passageway 82 length: | 8 mm to 30 mm, preferably between 8 mm and 20 mm, |
| passageway 82 diameter: | 0.5 mm to 6 mm, |

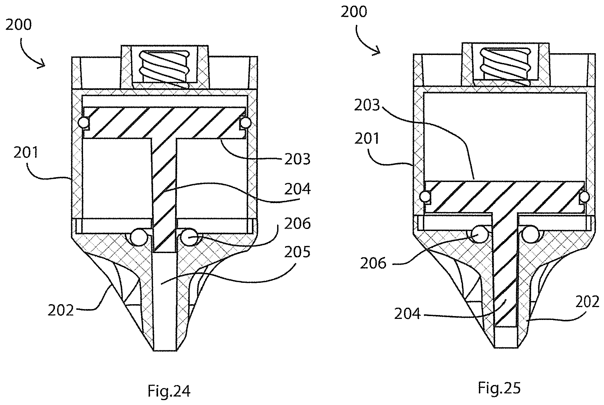

Referring to Figs. 24 and 25 an alternative container 200 has a chamber 201 and a cone 202. A piston 203 is on a stem 204 which moves in an axial passageway 205. In this case there is an O-ring 206 at the top of the cone 202 and surrounding the stem 204, to ensure leaks are avoided.

### Use of Device

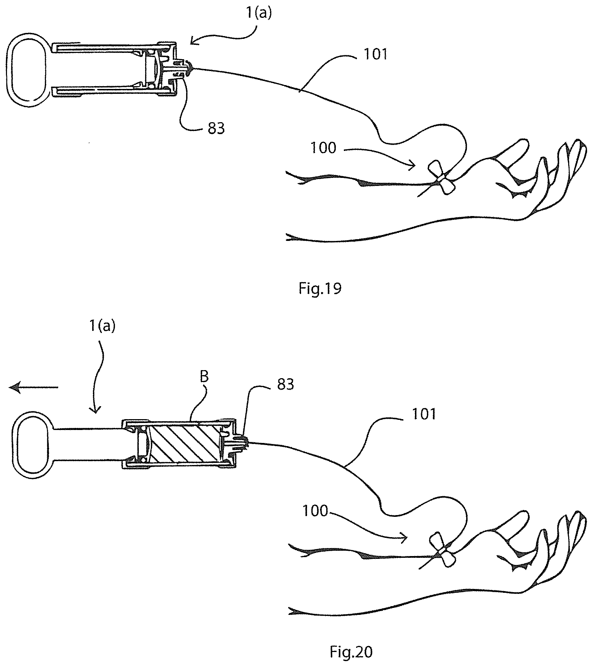

Referring to Figs. 19 and 20, the device is suitable to draw blood from a patient, by removing the container 1(b) and attaching a needle to the connector 21 at the luer-configured protrusion 83. By pulling the syringe piston 20 with the plunger 4 blood can be drawn into the syringe tube 3. The needle may comprise a butterfly needle 100 and tube 101. The blood is indicated by "B" in Figs. 19 and 20.

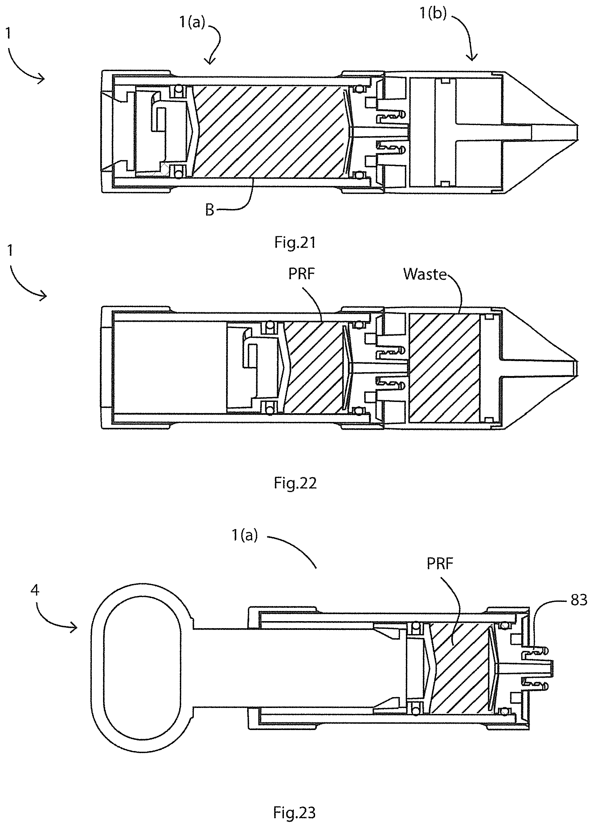

The plunger 4 is removed (Fig. 21), and the container 1(b) attached. The device is put in a centrifuge. The centrifugal force can be between 5000 and 50000 m/s² preferably between 10000 and 30000 m/s², and more preferably between 15000 and 20000 m/s². The time of the centrifugation is between 3 and 30 minutes, preferably between 5 and 15 minutes, and more preferably between 5 and 10 minutes. The protocol takes place within 1 to 60 minutes, preferably between 3 and 30 minutes, more preferably between 5 and 10 minutes after drawing the blood from the patient.

After the appropriate time and centrifugal force, the drawn blood B is separated to a platelet rich fibrin (PRF) fraction in the syringe tube 3, and red blood cells in the chamber 6 (Fig. 22).

The aperture 61, the gap 90, and the passageway 82 are fluid passages which play an important role in keeping formed fibrin in the syringe.

The centrifugal separation of blood components is already well known, with the use of anticoagulant, the two main blood separation products after centrifugation are plasma on the top and red blood cells (RBC) on the bottom. If anticoagulant is added and the whole blood is let to clot for at least 30 minutes and the whole blood is centrifuged afterwards, then it will separate to serum on the top and RBC on the bottom. If the whole blood is centrifuged immediately after drawing blood in a tube, which has a surface that activates the fibrin to fibrinogen conversion (e.g. glass, titanium, hydrophilic materials, anionic and cationic materials) then the whole blood will be separated to platelet rich fibrin on the top and RBC on the bottom. The RBC to whole blood ratio in all these blood separation methods is the same in one individual. This ratio is between 40% and 50%. With carefully choosing the ratio of container to syringe tube 3 the red blood cells are separated in the container from the serum fraction in syringe tube 3. This ratio is between 30% and 70%, preferably between 40% and 60%. During centrifugation the fibrinogen content of the whole blood contacts with the inner wall of the syringe tube 3 and the container, which activates the fibrinogen to fibrin conversion, thus polymerized fibrin gel (PRF) is formed in the upper part of the centrifuged whole blood, which is mainly in the syringe tube 3 after centrifugation. The formed PRF also encapsulates most of the platelet content of the whole blood, and as platelets contain biologically active proteins and growth factors these can be retrieved in the most convenient way by pressing the fluid out from the fibrin matrix.

After centrifuging, the detachable container 1(b) is removed and the PRF (or "SPRF", serum of Platelet rich fibrin) fraction can be administered by attaching the plunger 4 to the piston 20 (Fig. 23). The connector part 21(a) ensures that any formed gel network stays in the cylindrical syringe tube 3.

A needle can be attached with the luer-slip or luer-lock 83 to the connector 21, and the PRF fraction can be injected to the patient in one or more steps.

While blood separation due to the different densities of the blood ingredients is well known, surprisingly the device was suitable for both separating the blood fractions and for forming a fibrin clot. Taking into account the size of the fluid passage 82 diameter of 2 mm, the device still allowed the separation and did not interfere with the conversion of fibrinogen to fibrin. In order to both press out and separate the serum fraction from the fibrin matrix the gap 90 enables the passage of serum outwards. The upper surface of disc 60 is coated with a fibrin film from the fibrin matrix during pressing the serum outward and can stuck into passageway 61. Thus the dish 60 selectively filters fibrin but the gap 90 still enables fluid passage. The gap 90 is a closed space which may be utilized as a holder for certain activators, filters, and/or additives.

It will be appreciated that the device allows a blood sample to be taken in a conventional manner once the container 1(b) is removed. The container 1(b) may then be very easily re-attached using the screw threads. The passageway 82 provides a permanent link for blood/blood fraction flow between the tube 3 and the chamber 6 during centrifuging. It is suction from the piston 20 which pulls blood into the tube 3 from the patient. However, once being centrifuged the blood splits into its fractions with the major fractions being as described above. During centrifuging the piston 22 within the chamber 6 moves down under the forces of blood entering the chamber 6 and the centrifugal force. The piston 22 enables enhanced phase transfer, and reduces the air in the syringe 1(a) part. Without the piston 22, some of the air in the waste chamber 6 would migrate into the chamber 3 and would therefore make the injection less accurate.

Hence, the red blood cell containing fraction may be physically removed by un-screwing the container 1(b). The serum from platelet rich fibrin (PRF) may be administered to a patient upon connection of a needle to the connector 21, or it may be stored.

In other embodiments, the space 90 between the parts 21 (a) and 21 (b) can be utilized for the storage of reactive particles, for example titanium or glass beads.

Also, the chamber 6 may also be coated with reactive material, as could the syringe tube 3.

The syringe can be partially filled with anticoagulant, or any biologically active ingredient (e.g. antibiotics, recombinant growth factor, albumin, etc.) both before and after the centrifuging process and the whole system can be incubated as well. Pre-filling the syringe and incubating the device also allows the user to prepare both plasma and serum using the device.

Due to platelet rich fibrin (PRF) formation and squeezing out the serum from PRF the formed fibrin film may further be used as an autologous sealant.

We have identified the concentration interval of some growth factors that can be produced with the syringe and we have confirmed that there is no significant difference in these specific growth factors between the SPRF produced from the syringe and the SPRF produced from a glass tube.

With reference to the alternative container 200 of Figs. 24 and 25, upon pressure being applied by the centrifuge, the waste piston will move downwards and come in contact with the O-ring 206. As pressure increases the force being applied to the O-ring 26 will also increase thus creating a better seal.

The maximum gap left between the waste piston 203 and the waste container 201 at the end of its travel will be 0.75mm. This will only affect the ratio of fluid between the waste container and syringe positively as this gap will correspond to the gap/air pocket that exists around the spacer of the syringe when extracting blood.

The invention is not limited to the embodiments described but may be varied in construction and detail. For example, the connector first and second parts may be integral. The material of blood-contacting parts may be glass, but could alternatively be other such as plastics. Depending on the material type the clotting may be slower or faster, the device may be used with anticoagulated blood, in this case it may be used for plasma separation.

Some or all parts of the device may be coated to provide internal surfaces to assist activation leading to the formation of PRF. The coatings may be on a base material such as PMMA (poly (methyl methacrylate), PC (polycarbonate), PEEK (polyether ether ketone), PET (polyethylene terephthalate) which are applicable in those parts of medical devices that will be in contact with the drawn blood fractions can also start the clotting process upon contact. The effect of adding a coating is that the surface becomes more hydrophilic. This effect can be enhanced with the addition of hydroxyapatite as a thin inner film coating. Another possible approach is modifying the surface of the polymer using ionization due to the treatment of the surface with plasma phase ionizing materials such as air or O₂.

This coating is preferably applied to the inner side of the syringe, which comes in contact with the drawn blood. Thus, in one embodiment the glass tube (3) is replaced with a coated polymer and may be united with the casing (2) and distal connector (80), which gives the advantage of an easier and yet effective assembly of the device.

## Claims

1. A blood handling device comprising:
a syringe (1(a)) with a syringe tube (3) for taking a blood sample, and
a container (1(b)) releasably connected to the syringe, with a chamber (6) and a fluid passageway (61, 82) between the syringe and the chamber (6),
a connector (21) interfacing between the syringe (1(a)) and the container (1(b)),
said connector having said fluid passageway (61, 82), and
said connector comprises a first part (21(a)) forming a base for the syringe, and a second part (21(b)) arranged to connect to the container (1(b)) or to a cannula, wherein the syringe tube (3) and the container have an inner wall configured to activate fibrinogen to fibrin conversion, and thus to separate blood fractions and to form a fibrin clot upon contacting said blood sample with said tube (3) and said container during centrifugation.

2. The blood handling device as claimed in claim 1, wherein the syringe has a plunger (4) releasably connected to a piston (20) within the syringe tube (3).

3. The blood handling device as claimed in claim 2, wherein the plunger (4) and the piston (20) have inter-engaging screw threads (40,50).

4. The blood handling device as claimed in any preceding claim, wherein there is a gap (90) between said connector parts (21(a), 21(b)), and optionally said gap is in the range of 0.1 mm and 7 mm.

5. The blood handling device as claimed in any preceding claim, wherein the connector first part (21(a)) comprises a plate (60) with a surface facing into the syringe volume.

6. The blood handling device as claimed in claim 5, wherein the plate (61) is dish-shaped with a generally concave surface facing into the syringe volume.

7. The blood handling device as claimed in claims 5 or 6, wherein said plate (60) has an aperture (61) aligned with a passageway (82) in the container second part.

8. The blood handling device as claimed in claim 7, wherein said passageway is elongate.

9. The blood handling device as claimed in claims 7 or 8, wherein a gap (90) is maintained by spacers (63) of one part engaging in apertures (84) of the other part.

10. The blood handling device as claimed in any preceding claim, wherein the connector (21) first (21(a)) and second (21(b)) parts are releasably engaged.

11. The blood handling device as claimed in any preceding claim, wherein the connector (21) second part (21(b)) comprises a luer fastener (83) suitable for engaging a cannula or the container (5).

12. The blood handling device as claimed in any preceding claim, wherein the container (1(b)) has a piston (22) fitted to move in the chamber (6) in a direction away from the syringe under applied forces during centrifuging of the device.

13. The blood handling device as claimed in claim 12, wherein the chamber piston (22) has a lower stem (72) arranged to move axially within a guide passageway (73) in the container.

14. The blood handling device as claimed in claim 13, wherein the container (201) has a resilient seal (206) around said guide passageway at a bottom wall of the chamber.

15. The blood handling device as claimed in any preceding claim, wherein the width of the passageway is between 0.5 mm and 6 mm and the length of the passageway is between 8 mm and 30 mm.

16. The blood handling device as claimed in any preceding claim, wherein the device includes a hydrophilic blood-contacting material such as hydroxyapatite or a polymer with a plasma phase ionic surface modification.

## Patentansprüche

1. Blutbehandlungsvorrichtung, umfassend:
eine Spritze (1(a)) mit einem Spritzenrohr (3) zum Entnehmen einer Blutprobe, und
einen Behälter (1(b)), der lösbar mit der Spritze verbunden ist, mit einer Kammer (6) und einem Fluiddurchgang (61, 82) zwischen der Spritze und der Kammer (6),
einen Verbinder (21), der eine Schnittstelle zwischen der Spritze (1(a)) und dem Behälter (1(b)) bildet,
wobei der Verbinder einen Fluiddurchgang (61, 82) aufweist, und
wobei der Verbinder einen ersten Teil (21(a)), der eine Basis für die Spritze bildet, und einen zweiten Teil (21(b)), der angeordnet ist, um mit dem Behälter (1(b)) oder mit einer Kanüle zu verbinden, umfasst,
wobei das Spritzenrohr (3) und der Behälter eine Innenwand aufweisen, die konfiguriert ist, um eine Umwandlung von Fibrin in Fibrinogen zu aktivieren und somit, um Blutfraktionen zu trennen und um ein Fibringerinnsel zu bilden, wenn die Blutprobe während einer Zentrifugation mit dem Rohr (3) und dem Behälter in Kontakt kommt.

2. Blutbehandlungsvorrichtung nach Anspruch 1, wobei die Spritze einen Druckkolben (4) aufweist, der innerhalb des Rohrs (3) lösbar mit einem Hubkolben (20) verbunden ist.

3. Blutbehandlungsvorrichtung nach Anspruch 2, wobei der Druckkolben (4) und der Hubkolben (20) ineinandergreifende Schraubgewinde (40, 50) aufweisen.

4. Blutbehandlungsvorrichtung nach einem vorstehenden Anspruch, wobei zwischen den Teilen (21(a), 21(b)) des Verbinders ein Spalt (90) vorhanden ist und gegebenenfalls der Spalt in dem Bereich von 0,1 mm bis 7 mm liegt.

5. Blutbehandlungsvorrichtung nach einem vorstehenden Anspruch, wobei der erste Teil (21(a)) des Verbinders eine Platte (60) mit einer in das Spritzenvolumen hinein weisenden Oberfläche umfasst.

6. Blutbehandlungsvorrichtung nach Anspruch 5, wobei die Platte (61) schalenförmig ist, mit einer im Allgemeinen konkaven Oberfläche, die in das Spritzenvolumen hinein weist.

7. Blutbehandlungsvorrichtung nach Anspruch 5 oder 6, wobei die Platte (60) eine Öffnung (61) aufweist, die mit einem Durchgang (82) in dem zweiten Teil des Behälters ausgerichtet ist.

8. Blutbehandlungsvorrichtung nach Anspruch 7, wobei der Durchgang länglich ist.

9. Blutbehandlungsvorrichtung nach Anspruch 7 oder 8, wobei der Spalt (90) durch Abstandshalter (63) eines Teils aufrechterhalten wird, die in Öffnungen (84) des anderen Teils eingreifen.

10. Blutbehandlungsvorrichtung nach einem vorstehenden Anspruch, wobei der erste (21(a)) und der zweite (21(b)) Teil des Verbinders (21) lösbar in Eingriff stehen.

11. Blutbehandlungsvorrichtung nach einem vorstehenden Anspruch, wobei der zweite (21(b)) Teil des Verbinders (21) ein Luer-Befestigungselement (83) umfasst, das zum in Eingriff bringen einer Kanüle oder des Behälters (5) geeignet ist.

12. Blutbehandlungsvorrichtung nach einem vorstehenden Anspruch, wobei der Behälter (1(b)) einen Hubkolben (22) aufweist, der angebracht ist, um sich unter Kräften, die während des Zentrifugierens der Vorrichtung ausgeübt werden, in der Kammer (6) in einer Richtung weg von der Spritze zu bewegen.

13. Blutbehandlungsvorrichtung nach Anspruch 12, wobei der Kammer-Hubkolben (22) einen unteren Schaft (72) aufweist, der angeordnet ist, um sich innerhalb eines Führungsdurchgangs (73) in dem Behälter axial zu bewegen.

14. Blutbehandlungsvorrichtung nach Anspruch 13, wobei der Behälter (201) eine elastische Dichtung (206) um den Führungsdurchgang herum an einer Bodenwand der Kammer aufweist.

15. Blutbehandlungsvorrichtung nach einem vorstehenden Anspruch, wobei die Breite des Durchgangs zwischen 0,5 mm und 6 mm liegt und die Länge des Durchgangs zwischen 8 mm und 30 mm liegt.

16. Blutbehandlungsvorrichtung nach einem vorstehenden Anspruch, wobei die Vorrichtung ein hydrophiles blutkontaktierendes Material aufweist, wie beispielsweise Hydroxyapatit oder ein Polymer mit einer ionischen Oberflächenmodifikation in der Plasmaphase.

## Revendications

1. Dispositif de traitement du sang comprenant :
une seringue (1(a)) avec un tube de seringue (3) pour prélever un échantillon de sang et
un récipient (1(b)) raccordé de manière amovible à la seringue, avec une chambre (6) et un passage de fluide (61, 82) entre la seringue et la chambre (6),
un raccord (21) faisant interface entre la seringue (1(a)) et le récipient (1(b)),
ledit raccord ayant ledit passage de fluide (61, 82) et
ledit raccord comprenant une première partie (21(a)) formant une base pour la seringue et une seconde partie (21(b)) agencée pour se raccorder au récipient (1(b)) ou à une canule, dans lequel le tube de seringue (3) et le récipient ont une paroi interne configurée pour activer le conversion de fibrinogènes en fibrines et pour séparer ainsi des fractions de sang et former un caillot fibrineux lors de la mise en contact dudit échantillon de sang avec ledit tube (3) et ledit récipient au cours de la centrifugation.

2. Dispositif de traitement de sang selon la revendication 1, dans lequel la seringue a un plongeur (4) raccordé de manière amovible à un piston (20) à l'intérieur du tube de seringue (3).

3. Dispositif de traitement de sang selon la revendication 2, dans lequel le plongeur (4) et le piston (20) ont des filets de vis en prise (40, 50).

4. Dispositif de traitement de sang selon l'une quelconque des revendications précédentes, dans lequel il y a un intervalle (90) entre lesdites parties de raccord (21(a), 21(b)) et éventuellement ledit intervalle se situe dans la plage de 0,1 mm et 7 mm.

5. Dispositif de traitement de sang selon l'une quelconque des revendications précédentes, dans lequel la première partie (21(a)) du raccord comprend une plaque (60) avec une surface en regard du volume de la seringue.

6. Dispositif de traitement de sang selon la revendication 5, dans lequel la plaque (61) est en forme d'assiette avec une surface de forme générale concave en regard du volume de la seringue.

7. Dispositif de traitement de sang selon les revendications 5 ou 6, dans lequel ladite plaque (60) a une ouverture (61) alignée avec un passage (82) dans la seconde partie du récipient.

8. Dispositif de traitement de sang selon la revendication 7, dans lequel ledit passage est allongé.

9. Dispositif de traitement de sang selon les revendications 7 ou 8, dans lequel un intervalle (90) est maintenu par des espaceurs (63) d'une partie s'engageant dans des ouvertures (84) de l'autre partie.

10. Dispositif de traitement de sang selon l'une quelconque des revendications précédentes, dans lequel la première (21(a)) et la seconde (21(b)) partie du raccord (21) sont engagées de manière amovible.

11. Dispositif de traitement de sang selon l'une quelconque des revendications précédentes, dans lequel la seconde partie (21(b)) du raccord (21) comprend une attache luer (83) convenant pour s'engager sur une canule ou sur le récipient (5).

12. Dispositif de traitement de sang selon l'une quelconque des revendications précédentes, dans lequel le récipient (1(b)) présente un piston (22) monté pour se déplacer dans la chambre (6) dans une direction s'écartant de la seringue sous l'effet de forces appliquées au cours de la centrifugation du dispositif.

13. Dispositif de traitement de sang selon la revendication 12, dans lequel le piston (22) de la chambre a une tige inférieure (72) agencée pour se déplacer axialement dans un passage de guidage (73) du récipient.

14. Dispositif de traitement de sang selon la revendication 13, dans lequel le récipient (201) a un joint étanche élastique (206) disposé autour dudit passage de guidage sur une paroi inférieure de la chambre.

15. Dispositif de traitement de sang selon l'une quelconque des revendications précédentes, dans lequel la largeur du passage se situe entre 0,5 mm et 6 mm et la longueur du passage se situe entre 8 mm et 30 mm.

16. Dispositif de traitement de sang selon l'une quelconque des revendications précédentes, dans lequel le dispositif comprend un matériau hydrophile venant en contact avec le sang, tel qu'une hydroxyapatite ou un polymère avec une modification de surface ionique de la phase plasmatique.
